**Europäisches Patentamt**

**European Patent Office**

**Office européen des brevets**

(19)

(11) Publication number : **0 482 035 B1**

## (12) EUROPEAN PATENT SPECIFICATION

(45) Date of publication of patent specification :
**12.10.94 Bulletin 94/41**

(51) Int. Cl.⁵ : **G01N 3/56,** G01N 15/06

(21) Application number : **90910526.4**

(22) Date of filing : **06.07.90**

(86) International application number :
**PCT/NO90/00112**

(87) International publication number :
**WO 91/00991 24.01.91 Gazette 91/03**

(54) **SAND DETECTOR.**

(30) Priority : **07.07.89 NO 892819**

(43) Date of publication of application :
**29.04.92 Bulletin 92/18**

(45) Publication of the grant of the patent :
**12.10.94 Bulletin 94/41**

(84) Designated Contracting States :
**AT BE CH DE ES FR GB IT LI NL SE**

(56) References cited :
**EP-A-00 166 39**
**EP-A-03 173 39**
**US-A- 3 767 916**

(73) Proprietor : **NORSK HYDRO TECHNOLOGY B.V.**
**P.O. Box 2**
**NL-4540 AA Sluiskil (NL)**

(72) Inventor : **SONTVEDT, Terje**
**Söndre Gjettum 58**
**N-1346 Gjettum (NO)**

EP 0 482 035 B1

## Description

The invention relates to a method of measuring the amount of abrasive material in a flow of liquid and/or gas. The method can for example be employed to supervise the production rate of sand in an oil and/or gas production well.

Production of oil and gas often contains sand. For production wells in a sandstone reservoir without a sand controlling system it is assumed that more than 25 ppm of quartz particles with diameter of 1-1000 um may be produced. If a sand controlling system is applied the sand production will be approximately 25 ppm with a diameter of 0-100 $\mu$m. Sand production can not only cause severe erosion of the production equipment, but large quantities of sand can also be accumulated in the equipment and result in production break down. If the production of sand are measured reliably, the production rate can be adjusted and the problems related to erosion and accumulation of sand can be reduced. The maximum production rate without producing sand can thus be established. It is therefore a need to monitor small/medium sand production rates with either small particles (applying gravel packs) or with full spectrum of the particle size. Monitoring should preferably be applied on each individual well. There are also various other applications where continuous supervision is important when there is danger of contamination of abrasive materials in the fluid flow.

Different systems have been used or suggested for monotoring or detecting sand content in a fluid well. One system to apply in a fluid flow to determine the abrasion caused by sand is the erosion probe. Sand can erode for example a thin, hollow-walled probe which is mounted in the fluid flow. The pressure difference between the fluid flow and a reference point will activate an alarm. The probe will therefore have a considerable time delay before it detects sand and it will not give continuous monotoring of the sand content in the fluid flow or of the sand production rate.

There are also known different acoustic probes that either can be clamped on the outside of the pipewall or mounted inside the pipe. This probe can detect sand production in either a gas or a liquid flow. The ability to distinguish between sand noise and other noise is not satisfactory in the intermittent or anular/mist fluid flow. The calibration of the acoustic probe has to be performed with real production parameter and by injection of sand. The calibration will change when the production rate or other sources of noise are varied.

Small particles (0-0,5 mm dia.) produce acoustic energy too low to discriminate between particle- and flow noise. One such acoustic probe is described in NO patent No. 140.838.

US patent No. 3.678.273, US patent No. 3767916 and EP patent No. 0317339 discloses various methods for measuring erosion caused by abrasive fluid. Common for this inventions are that a detector coated with a radioactive material is positioned in a fluid flow, for example oil slurry. A detector are activated by the radiation from the radioactive coating due to the thickness reduction caused by the abrasive fluid. The detector is in contact with a control- and monotoring unit on the outside of the pipe. The particle concentration of the flow is determined by determining the particle concentration of an equivalent flow of known composition which causes the measured reduction of radiation emission when it contacts an equivalent material at the measured flow velocity. The particle concentration in the flow can thus be estimated. The detector disclosed in said patents is mounted in the flow and will thus be an obstruction to the flow. The detector will not be able to detect small particles because these particles will follow the flow and pass around the obstruction. This detector can therefore not be used in a high pressure hydrocarbon pipe where it is impossible to predict the sand distribution. The accuracy and how the particle content are measured are not discussed. The method requires calibration and a uniform distribution of the abrasive particles.

The object of the present invention is to provide an improved method for continuous monotoring of the production rate in a hydrocarbone well. It is important to be able to estimate the total mass of sand produced without having to calibrate the system. It is also an object to provide an accurate system which is sensitive to a low production rates and also be able to adjust the sensitivity of the probe to the necessary limits for an adequate control. The system has to tolerate a two phase flow and detect small particles.

This and other object is achieved by the method defined by the present claims.

By the present invention one has achieved a method to detect abrasive material in a production flow containing unknown amount and distribution of particles, without having to calibrate the system. Several probes are activated and emitts radiation to a detector at the outside of the pipewall. Each monitored probe emitts signals only to one detector. The probes are positioned with a distance inbetween each other both in the longitudinal direction and around the circumference. The probes are positioned with an angle $\alpha$ to the fluid flow where $20° < \alpha < 60°$. Without any signal processing, the probes will registrate that the fluid flow contains particles if the thickness of the radioactive coating is reduced by 0,1%. The particle concentration is determined if the thickness of the coating is reduced by 0,25%.

The quantity Mg of particles impinging the probes can be determined by the following equation

$$Mg = \frac{E\,A}{K\,F\,V^{2,62}} \text{ (kg)}$$

where

E      = Reduction of probe thickness (mm)

A      = The pipe cross sectional area (m$^2$)

K      = Material constant for the activated material

F      = Function of the flow density and mean diameter Dp (mm) of the solid particles in the reservoir;

$$F = \frac{Dp}{B^{\,0,5}}$$

B      = Constant for all gas/liquid ratios

        = Flow density (kg/m$^3$).

V      = Velocity of the flow (m/s)

The invention will be explained in detail with reference to the enclosed drawings where:

Fig. 1          illustrates the test set up.

Fig. 2          illustrates a section of the test set up including a detector and analyser.

Fig. 3          shows the erosion velocity as a function of the angle to the test specimens in the flow, fluid flow velocity 6 m/s.

Fig. 4          shows the erosion velocity as a function of the test specimens angle to the flow, at fluid flow velocity of 3 m/s.

Fig. 5A          shows the erosion velocity as a function of the distance from the test specimen to the center line of the test tube. Fluid flow velocity is 6 m/s.

Fig. 5B          illustrates the erosion velocity as in 5A, but the fluid flow velocity is 4,5 m/s.

Fig. 6          illustrates the optimum location of three probes.

Fig. 7A          shows the erosion velocity as a function of sand concentration.

Fig. 7B          illustrates the correlation between the flow velocity and erosion velocity.

Fig. 8          shows the erosion velocity for steel as a function of the flow velocity for quartz particles in a flow of hydrocarbons.

Fig. 9          illustrates the loss of material in a fluid flow containing 2300 ppm sand during two hours.

Fig. 10          illustrates the loss of material in a fluid flow containing 400 ppm sand during six hours.

The sand detector is based on measuring the radioactive material loss. The measurement of material loss by a radioactive technique is based on irridiating the test piece with neutrons or other energy rich particles, forming radioactive isotops in the test specimen. The radioactive isotops will emitt radioactivity and simultaneously be transformed to a new material. The intensity of the emitted radiation can be measured and will be directly proportional to the amount of material in the specimen. Because that the test speciemen corrodes or erodes the intensity will be reduced due to the material loss, and thus it will be possible to measure the corrosion or erosion very accurately.

The specimens used to measure the material loss can be made radioactive by thin coat activation or neutron activation. In both cases the speciemens are irradiated with energy rich particles forming radioactive isotopes in the material. The actual quantity isotopes formed is less than 1 ppm. The material properties are not changed. Neutron activation implies neutrons with deep penetration depth causing most of the irradiation to pass through the material. The activating profile has an almost constant activation level. It is therefore relatively easy to interpret the date because the loss in irradiation intensity is a direct expression of the weight loss of the specimen. When it is activated, it is important to form isotops having a -energy large enough to reach the detector. It is also important to use isotops with a half-life for continuous erosion-/corrosion measurement over a period of several years.

An applicable isotop Co$^{60}$ is formed when steel is neutron activated, because almost all steels contain small amounts of Co. The Co$^{60}$ isotope has a -energy (1,17 MeV and 1,32 MeV) and the half-life is 5,27 years. With this relatively long half-life one can perform erosion-/corrosion measurements on process equipment for a period of 20-25 years without changing the specimens or penetrate the equipment.

However, during the experiments it was used a stamped out circular 110 um thick Inconell 600 steel folie which under irradiation mainly emitted the isotop $51_{Cr}$ and some $60_{Co}$. $51_{Cr}$ has short half-life and low -energy (0,32 Mev).

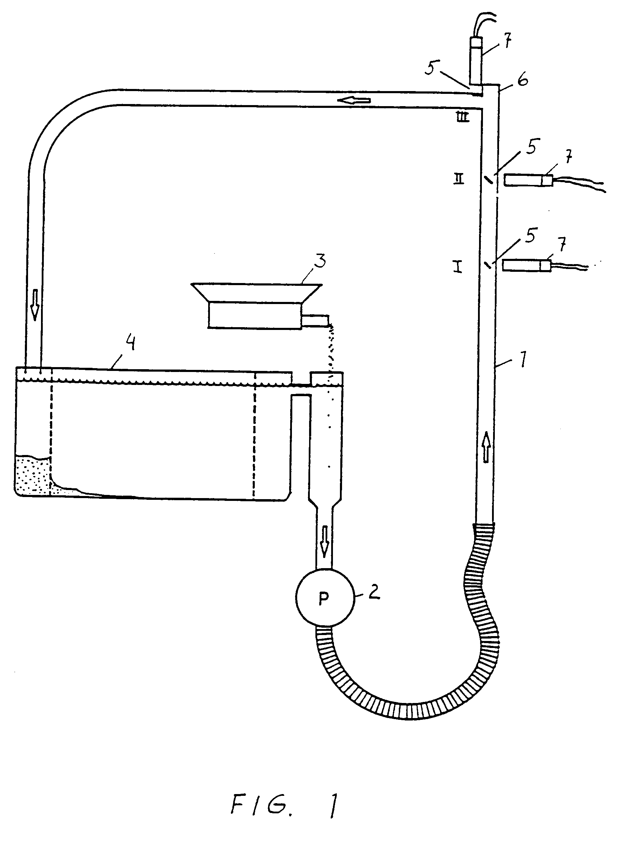

The experiments were performed by the test set up as shown in Fig. 1. It comprised a test section 1 of steel, a pump 2 with variable capacity, a sand feeder 3 with variable feeding rate, and necessary tanks 4 for sand and water. In the first part of the test period the sand was fed continuously without resirculation. The sand grains were of medium size, approx. 0,50 mm. The flow velocity could be regulated from 1 to 6 m/s and the sand flow from 1 to 30.000 ppm. The largest sand concentration used in the tests was 5.400 ppm.

The test specimens 5 was positioned as shown in the figures. Three different sizes of test spesimes were used. One where the activated folio was positioned at the end of the pipe 6, one where the test piece was positioned in the vertical pipe 1 at an angle of 20° with respect to the fluid flow, and one where the activated folio was mounted in the middle of the pipe. The different locations is indicated on the drawings as I, II, III. The test specimens 5 were mounted on a movable axel so the angle between the activated surface and the fluid flow could be adjusted and be pushed/pulled inside the pipe. The detector is indicated by the number 7 on Fig. 1.

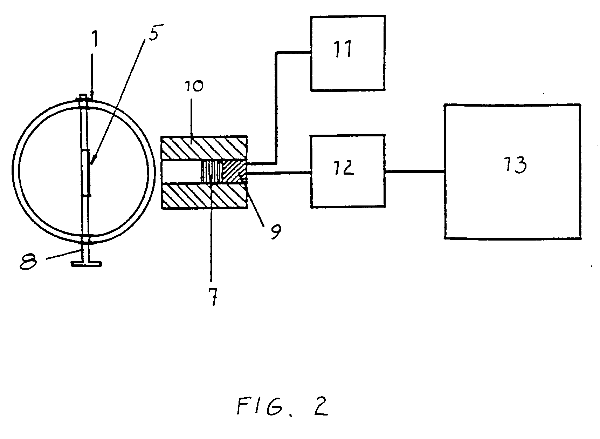

Fig. 2 is an enlarged section of the test upset illustrated in Fig. 1. The fluid flow passes the pipe 1. The activated test specimen 5 was mounted on an rotatable and movable arm 8. The detector 7 was positioned approx. 10 cm from the pipe. Increased distance from the pipe would require a stronger energy source because the irradiation intensity is reduced by the square of the distance. The thickness of the pipe wall can be larger if necessary or one can use a concrete cover isolation etc. between the test specimen and the detector. But the material between the test piece and the detector has to be constant and should not change over time. The gamma radiation from the surface was picked up by a scintilation detector with NaI-crystalls emitting light when radiated. The energy band of the light gave specific information of the radiation intensity from the surface of the test piece.

The detector 7 is connected to a pre-amplifier 9 and protected by a lead cover 10. It was also connected to high voltage 11, an amplifier 12 and an analyser 13. Most of the experiments were performed with two of the movable test pieces positioned one after the other with a distance of 60 cm.

Several tests were performed to get the optimum conditions for the process.

Test 1.

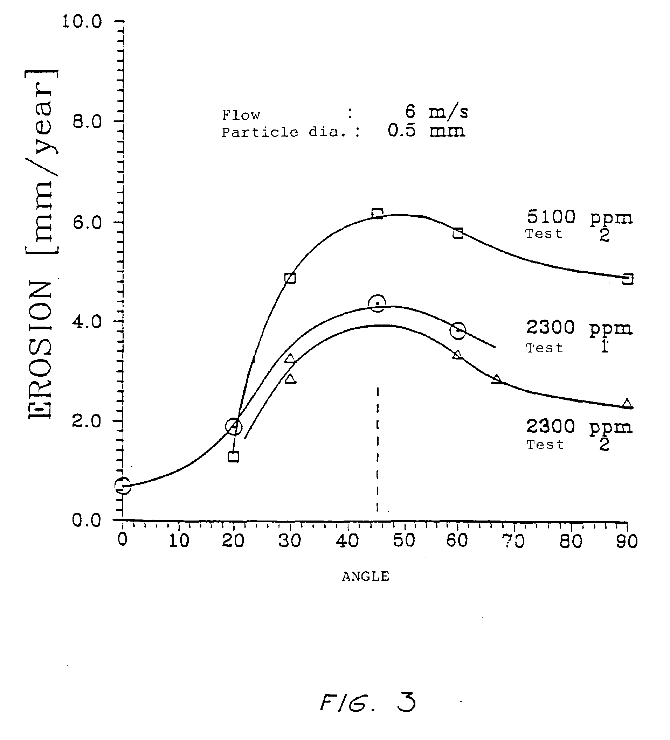

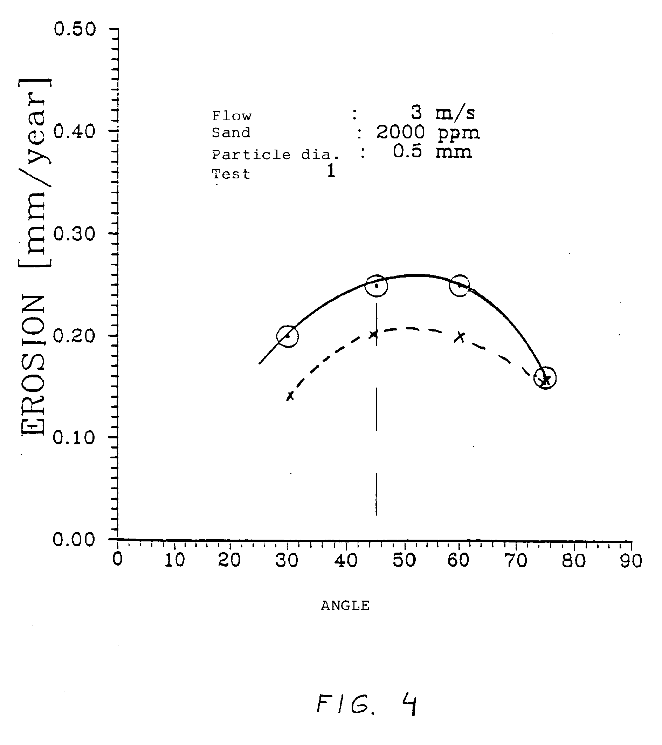

Tests were carried out to examine the effect of the test specimens angle with respect to the fluid flow. Movable test specimens were used. Fig. 3 illustrates the erosion velocity as a function of the angle to the test specimens, at fluid velocity of 6 m/s and sand concentration of 5.100 ppm and 2.300 ppm. The angle between the probes and the flow varied between 0 and 90° and the probes location was in the middle of the pipe. From Fig. 3 one can conclude that the erosion velocity increases when the angle is increased up to 45°, than decreases and reaches a minimum at 90°. In Fig. 4 the erosion velocity is illustrated with a reduced flow velocity of 3 m/s. The test results indicates that the maximum erosion rate is at approx. 50°, then decreases down to a minimum at 90° which is at a level below the results from the test when the velocity was 6 m/s. The dotted line indicates the calculated values.

Test 2.

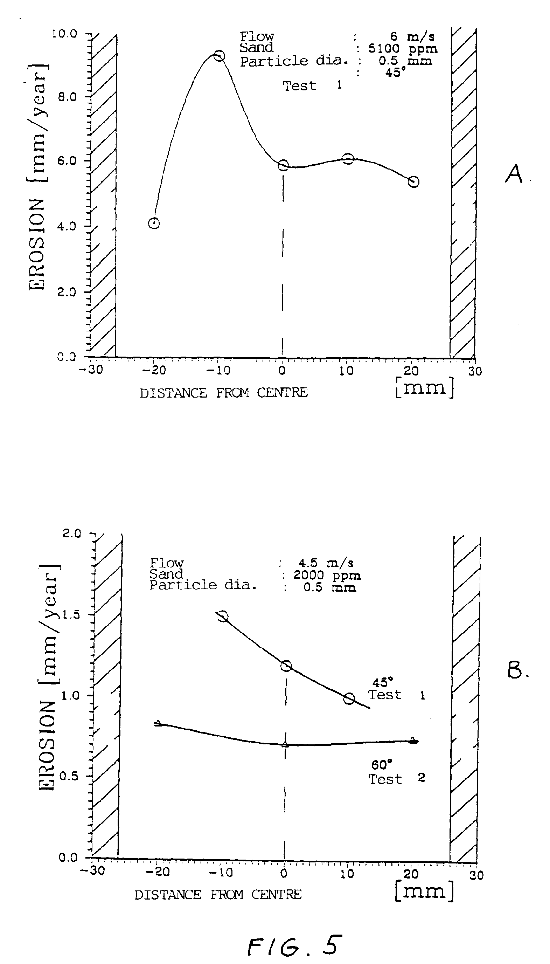

In several test the test specimens where moved backward and forward transversally to the flow direction to study the sand distribution. Fig. 5A illustrates the results from test specimen I from the test with a flow velocity of 6 m/s, angle 45°, sand concentration 5.100 ppm and sand grain size 500 um. The graph indicates a non-uniform distribution in the pipe. The inlet length was 2,5 m. Fig. 5B illustrates the same type of test as described above, but with a flow velocity of 4,5 m/s, sand concentration 2.000 ppm and a test specimen angle of 45° and 60° for specimen I and II. The sand distribution is non uniform for specimen I with respect to the central axis in the pipe. The sand distribution is more uniformly distributed for specimen II. This indicates that specimen I which is mounted 60 cm in front of specimen II was located in a more homogenious sand/water flow.

For location of the test specimen as shown in Fig. 1 the largest erosion on the specimen was measured in the centre of the pipe and with an angle of 45° to the flow direction (test I). The erosion on the flush mounted specimen 1 cm from the end of the T-shaped pipe was 60% of the erosion on test specimen III. Test specimen III mounted at the wall had an erosion 75% of test specimen I. This indicates that sand is concentrated in the centre of the pipe.

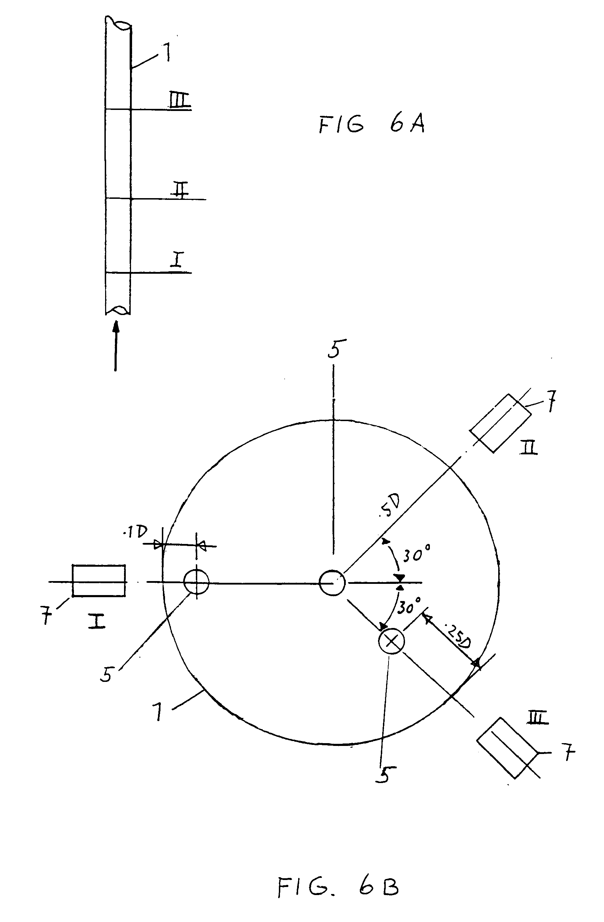

The sand distribution and the fluid flow pattern in a hydrocarbon flow is often unpredictable. The variations in the flow pattern also change the sand production and distribution in the pipe. It is therefore necessary with more than one specimen to monitore the total sand production. Figures 6A and B illustrate examples of an optimal location of three specimens 5 in a pipe 1. This location is optimal for a flow dominated either by liquid or gas. Specimen 5 and the detector 7 are located on three different levels, I,II,III as shown in Fig. 6A. The gap between the different detectors were adequate to allow only one test specimen to send signals to one detector, ie. a distance of 0,5 - 1 m between the detectors. The tests showed that the location of the specimen registrated the total particle content in the above described flow. It will however always be necessary to have one specimen in the middle of the process flow and one or more at the side of the flow to cover and detect a non-uniform flow. The test specimen were mounted with an angle of 45° to the process flow.

### Test 3.

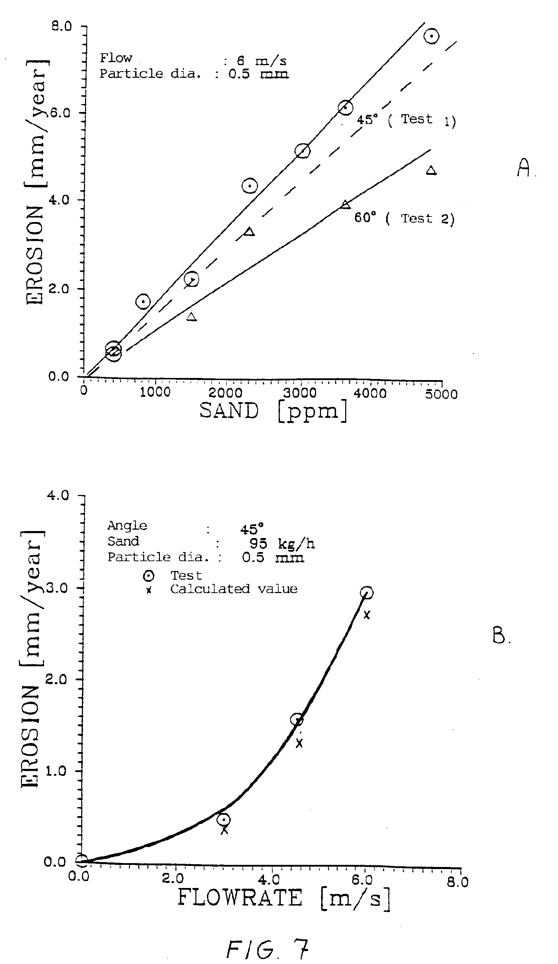

Tests were carried out to determine the effect of the sand concentration in the process flow. Fig. 7A illustrates the erosion velocity as a function of sand concentration when the angle of the test specimen to the process flow was 45° and 60°, and the fluid flow velocity 6 m/s. The grain size of the sand was 0,5 mm. As the graph illustrates, it is a linear correlation between the erosion velocity and the sand concentration. The scatter of some tests are due to tests taken from various test series. The dotted line is the calculated value.

### Test 4.

The effect of the flow velocity on the erosion velocity were determined. This is illustrated in Fig. 2B. The tests were carried out with a test specimen located with an angle of 45° to the flow, the sand concentration 2.000 ppm and the grain size of the sand 500 um. When the flow velocity increases, the sand concentration passing a section of the pipe increases proportionally. The erosion velocity measured at 3 m/s and 4,5 m/s was therefore corrected to represent the mass of sand equal to 95 kg/h passing the test specimen. The erosion velocity increases exponentially with the flow velocity. The dotted curve shows the calculated erosion velocity when the velocity increases with a fluid flow velocity raised to the power of 2,3 (which is relatively common for sand in gas flow).

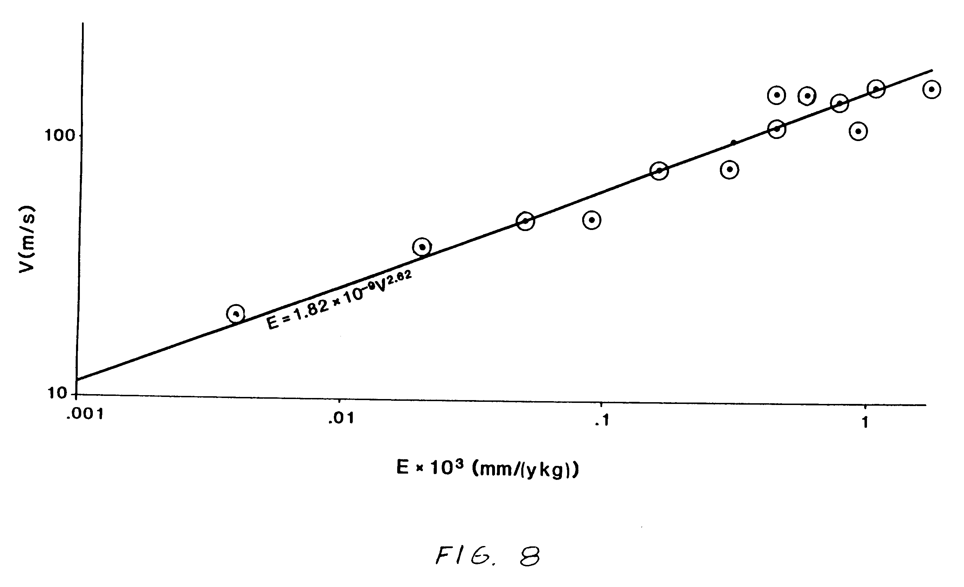

The inventor also performed different tests in three phase flow (gas/liquid/sand flow) to find the relationship between the changes in the thickness to the probes and the concentration of particles. The results were obtained by measuring the weight loss and using stationary ultra sound probes. The litterature are reporting test results where erosion velocity as a function of flow velocity has been investigated for different steel qualities, particles and particle size in various environment. The erosion depends on the shape and hardness of the particle. From the reported data, 17 data were chosen for erosion on steel caused by semi angular quartz sand with a distribution in size equal to sand produced in an oil/gas well. In Fig. 8 the data are plotted in a graph showing the erosion velocity as a function of flow velocity.

The results verify that it is a unique relation between the change in thickness and sand concentration. The quantity of solid particles Mg (sand etc.) which has impinged an radioactive surface is given by:

$$Mg = \frac{E\,A}{K\,F\,V^{2,62}} \text{ (kg)}$$

where

E = Reduction of the probe thickness (mm)
A = The pipe cross sectional area (m$^2$)
K = Material constant for the activated material
F = Function of the flow density and mean diameter Dp (mm) of solid particles in the reservoir;

$$F = \frac{Dp}{B^{0,5}}$$

B = Constant for all gas/liquid ratios
= Flow density (kg/m$^3$).
V = Velocity of the flow (m/s)

The calculated values are given by the dotted line in Fig. 4 and 7.

The sand concentration can be calculated by registrating the material loss for the probes in the process flow.

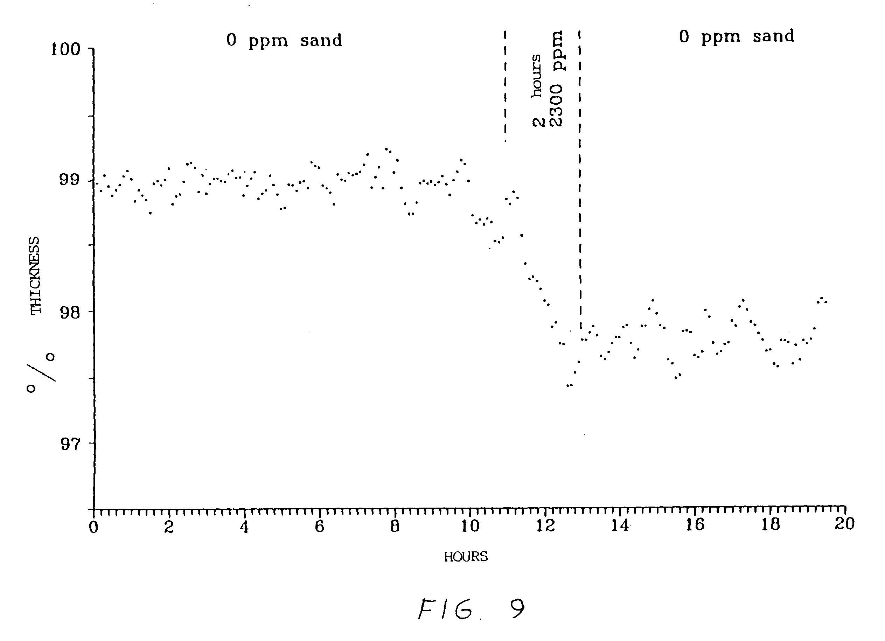

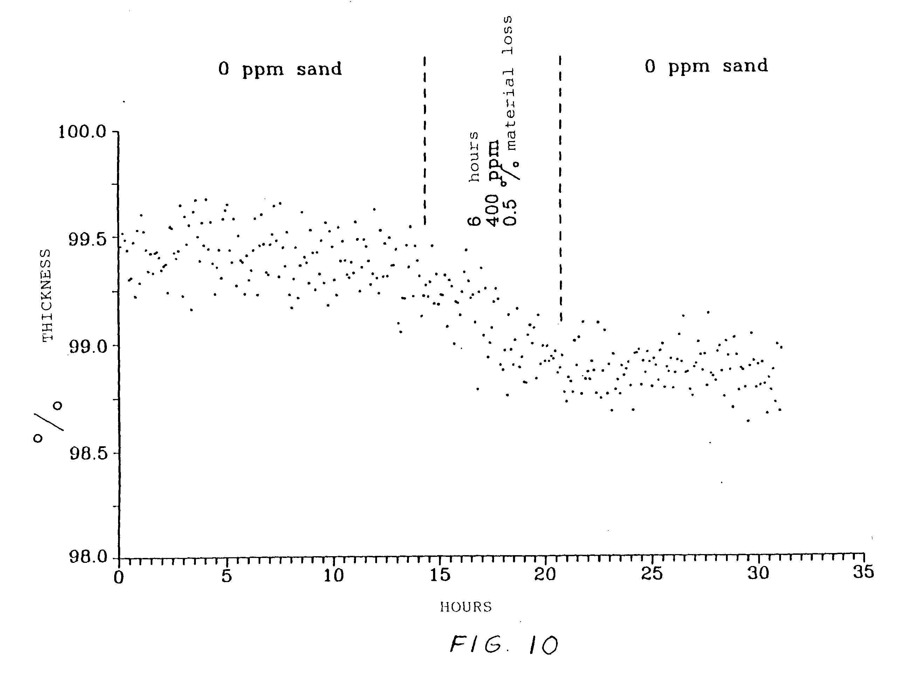

Fig. 9 shows the material loss during two hours at a sand concentration of 2300 ppm relative to the fluid flow. The test specimens had an angle of 45° relative to the fluid flow and the material loss was 1,05%. This is clearly indicated in the figure. Addition of 400 ppm sand for 6 hours gave also a clear indication as seen from Fig. 10. The loss of material was 0,5%. A material loss of 0,2% in 6 hours is also visible. The sensitivity of the equipment depends on how the data are sampled and analyzed. A material loss of 0,1% can be measured if the data are recorded and analyzed after the test. The data given in Fig. 9 and 10 are not analyzed, but by using more sofisticated statistics the accuracy of the data analyzed can be improved and the time delay before the sand rate are detected can be further reduced.

It was found that without analyzing the signal, a reliable indication of sand production is at a 0,1% thickness reduction of the specimens and the total sand concentration is confirmed to be at 0,25% of the thickness reduction. By signal analyses the corresponding numbers are 0,05% and 0,1%. The accuracy for the system can be varied by changing the thickness of the activated specimens, very thin specimens give a high level of accuracy. Specimens with different thickness can be used depending on what life length is desired for the specimens, and how important a fast response regarding the particle content is.

The method will detect sand production in a gas- or oil well in the following way:

EXAMPLE 1

It was used a gold specimen with cobolt as the gamma radiation source. The thickness was 0,01 mm and it was mounted 45° to the process flow. The following conditions shown in table 1 are for a gas well;

## Table 1 - Gas well

|  | start prod. | last year prod. |
|---|---|---|
| Allowable sand production (kg/day) | 28 | 9 |
| Allowable fines production (kg/day) | 137 | 42 |
| Velocity well head (m/s) | 11 | 21 |
| Measured sand prod. after (hours) kg) | 2,3 (3 kg) | 1,4 (0,8 |
| Calculated particle prod. rate within (hours) kg) | 6,3 (7 kg) | 3,5 (1,3 |

The velocity in a gas well is high. Due to the danger of severe erosion in the well head only low concentration of sand is allowable. Sand production will be detected when only 0,5 to 2,5 kg sand has been produced.

EXAMPLE 2

The same type of test specimens as in Example 1 were used in an oil producing well having following flow conditions as listed in table 2.

## Table 2 - Oil well

| Allowable sand prod. (kg/day): | 1351 (13% chrom steel) |
|---|---|
| Actual sand prod. (kg/day): | 742 |
| Velocity at well head (m/s): | 3,9 |
| Measured sand prod. after (hr) | 1,8 ( 99 kg) |
| Calculated particle prod. rate after (hr) | 4,5 (430 kg) |

The sand production in this well was 760 kg/day when the flow velocity was 3,9 m/s (gas flux is 1,6 $m^3/s/m^2$). Because of low production rate and gas/liquid ratio in this example it is difficult to detect sand for all detection system based on the particles energy. But by the method according to the invention it is possible to state that sand is produced after 2 hours of measurements and with a sand production of 100 kg. Total sand production can be calculated after 4,5 hours with an accumulated sand production of 430 kg. If further accuracy is required, the probes can be mounted in a pressure reducer (choke), provided that is a part of the process.

The tests illustrate that the system is independent of calibration. Even though the method according to the invention mainly has been described with regard to detection of sand in a hydro carbon flow, it is suitable

EP 0 482 035 B1

for detection of sand in other types of process flow where it is important to measure the concentration of abrasive particles. It is possible to detect particles as small as 0 - 0,05 mm in diameter. The sensitivity of the probes can be adjusted according to requirements. By selecting suitable thickness and material for the activated measuring element coated on the probe, measurements of sand concentration in oil wells (low sand particle energy) and gas well (high sand particle energy) can be accomplished with the required accuracy.

## Claims

1. A method of detecting the content of particulate material in a liquid and/or in a gas flow guided by a pipe by positioning at least one probe consisting of an abradable radioactive material in the fluid flow in the pipe and measuring the rate at which the emission of radiation is diminished by the abrasion of the radioactive material by a detector (7) mounted on the outside of the pipe guiding the fluid **characterized in that** the particle content Mg in the liquid and/or in the gas flow is detected by means of a plurality of said probes (5) mounted at different positions along the pipe and at different positions with respect to the cross section area of the pipe, each probe having one corresponding detector (7) arranged to detect the signals from the probes and in that the thickness of the probes are registered and used to calculate the particle content of the liquid and/or gas flow.

2. A method according to claim 1,
   **characterized in that**
   the probes are mounted with an angle $\alpha$ ($20° < \alpha < 60°$) to the direction of the process flow.

3. A method according to claim 2,
   **characterized in that**
   the angle $\alpha$ is approx. 45°.

4. A method according to claim 1,
   **characterized in that**
   probes (5) formed as sircular discs are used.

5. A method according to claim 1,
   **characterized in that**
   the concentration Mg of solid particles impinging the probes in a hydrocarbon flow is determined;

$$Mg = \frac{E\,A}{K\,F\,V^{2,62}}\ (kg)$$

where

| | |
|---|---|
| E | = Reduction of the probe thickness (mm) |
| A | = The pipe cross sectional area ($m^2$) |
| K | = Material constant for the activated material |
| F | = Function of the flow density and mean diameter Dp (mm) of solid particles in the reservoir; |

$$F = \frac{Dp}{B^{0,5}}$$

| | |
|---|---|
| B | = Constant for all gas/liquid ratios |
| | = Flow density ($kg/m^3$). |
| V | = Velocity of the flow (m/s) |

## Patentansprüche

1. Verfahren zum Ermitteln des Gehaltes an partikelförmigem Material in einem Strom einer Flüssigkeit und/oder eines Gases im Inneren einer Rohrleitung durch Anordnen von wenigstens einer aus einem abreibbaren radioaktiven Material bestehenden Sonde in dem besagten Strom in dem Rohr und durch Messen des Ausmaßes der Abnahme der Ausstrahlung von Radioaktivität infolge des Abriebes von radioaktivem Material mit Hilfe eines an der Außenseite des den besagten Strom führenden Rohres angeordneten Detektors (7), dadurch gekennzeichnet, daß der Gehalt Mg an Partikeln in den Gas- und/oder Flüssigkeitsstrom mit Hilfe einer Mehrzahl der besagten Sonden (5) festgestellt wird, daß die Sonden an verschiedenen Stellen entlang des Rohres und in unterschiedlichen Positionen in Bezug auf die Querschnittsfläche des Rohres angeordnet sind, wobei für jede Sonde ein dazugehöriger Detektor (7) zur Ermittlung der Signale der Sonden vorgesehen ist und daß die Dicke der Sonden aufgezeichnet wird und zur Berechnung des Partikelgehaltes in dem Flüssigkeits- und/oder Gasstrom benutzt wird.

2. Verfahren gemäß Anspruch 1, dadurch gekennzeichnet, daß die Sonden unter einem Winkel $\alpha$ von 20°< $\alpha$ < 60° in Bezug auf die Richtung des Prozeßstromes montiert werden.

3. Verfahren gemäß Anspruch 2, dadurch gekennzeichnet, daß der Winkel $\alpha$ ungefähr 45° beträgt.

4. Verfahren gemäß Anspruch 1, dadurch gekennzeichnet, daß Sonden (5) benutzt werden die als runde Scheiben ausgebildet sind.

5. Verfahren gemäß Anspruch 1, dadurch gekennzeichnet, daß in einem Kohlenwasserstoffstrom die Konzentration Mg an festen auf die Sonde auftreffenden Partikeln bestimmt wird durch:

$$Mg = \frac{E \cdot A}{K \cdot F \cdot V^{2,62}} \text{ (kg)}$$

wobei:

E = Abnahme der Sondendicke (mm)
A = Querschnittsoberfläche des Rohres ($m^2$)
K = Materialkonstante des aktivierten Materials
F = Funktion der Strömungsdichte und des mittleren Durchmessers Dp (mm) der festen Partikel in dem Speicher;

$$F = \frac{Dp}{B^{0,5}}$$

B = Konstant für alle Verhältnisse Gas/Flüssigkeit
= Strömungsdichte ($kg/m^3$)
V = Geschwindigkeit des Stromes (m/s)

## Revendications

1. Procédé de détection de la teneur en matière particulaire dans un écoulement de liquide et/ou de gaz guidé par une canalisation en disposant au moins une sonde constituée d'une matière radioactive susceptible d'être abrasée par l'écoulement de fluide dans la canalisation et en mesurant la vitesse à laquelle l'émission de la radiation est diminuée par l'abrasion de la matière radioactive à l'aide d'un détecteur (7) monté à l'extérieur de la canalisation guidant le fluide, caractérisé en ce que la teneur en particules Mg dans le flux de liquide et/ou de gaz est détectée à l'aide d'une série desdites sondes (5) montées à différents endroits le long de la canalisation et à différents endroits vis-à-vis de la section transversale de la canalisation, chaque sonde ayant un détecteur correspondant (7) agencé pour détecter les signaux provenant des sondes, et en ce que les épaisseurs des sondes sont enregistrées et utilisées pour calculer la teneur en particules du flux de liquide et/ou de gaz.

2. Procédé selon la revendication 1, caractérisé en ce que les sondes sont montées avec un angle $\alpha$ (20°< $\alpha$ < 60°) vis-à-vis de la direction du flux de traitement.

3. Procédé selon la revendication 2, caractérisé en ce que l'angle $\alpha$ est d'environ 45°.

4. Procédé selon la revendication 1, caractérisé en ce que l'on utilise des sondes (5) se présentant sous la forme de disques circulaires.

5. Procédé selon la revendication 1, caractérisé en ce que la concentration Mg de particules solides heurtant les sondes dans un flux d'hydrocarbures est déterminée comme suit :

$$Mg = \frac{E A}{K F V^{2,62}} \text{ (kg)}$$

où
E = réduction de l'épaisseur de la sonde (mm)
A = surface en coupe transversale de la canalisation ($m^2$)
K = constante pour la matière activée
F = fonction de la densité du flux et du diamètre moyen Dp (mm) de particules solides dans le gisement

$$F = \frac{Dp}{B^{0,5}}$$

B = constante pour tous les rapports de gaz/liquide
Dp = poids spécifique du flux (kg/m3)
V = vitesse du flux (m/s)

FIG. 1

FIG. 2

Flow          :      6 m/s
Particle dia. :    0.5 mm

FIG. 3

FIG. 4

A.

B.

FIG. 5

1

III

II

I

FIG 6A

5

7

II

.5D

30°

.1D

7    I

30°

.25D

5

7

III

7

5

FIG. 6B

FIG. 7

FIG. 8

EP 0 482 035 B1

FIG. 9

EP 0 482 035 B1

FIG. 10